# EUROPÄISCHE PATENTSCHRIFT

(11) **EP 1 299 347 B1**
(45) Veröffentlichungstag und Bekanntmachung des Hinweises auf die Patenterteilung: **27.04.2005**
(21) Anmeldenummer: 01957948.1
(22) Anmeldetag: 10.07.2001
(51) Int. Cl.: C07C 231/24, C07D 201/16

(54) **VERFAHREN ZUR ABTRENNUNG VON AMMONIAK**
METHOD FOR SEPARATING AMMONIAC
PROCEDE DE SEPARATION D'AMMONIAC

(30) Priorität: 11.07.2000 DE 10033518
(43) Veröffentlichungstag der Anmeldung: 09.04.2003
(73) Patentinhaber: BASF AKTIENGESELLSCHAFT, 67056 Ludwigshafen (DE)
(72) Erfinder: LUYKEN, Hermann, 67069 Ludwigshafen (DE)
(86) Internationale Anmeldenummer: PCT/EP2001/007945
(87) Internationale Veröffentlichungsnummer: WO 2002/008171

(56) Entgegenhaltungen:
- WO-A-01/83441
- WO-A-95/14665
- WO-A-96/20923

## Beschreibung

Die vorliegende Erfindung betrifft ein Verfahren zur Abtrennung von Ammoniak (I) aus Mischungen (II), die erhältlich sind durch Umsetzung von Edukten (III) ausgewählt aus der Gruppe bestehend aus Nitrilen (IIIa), Aminen (IIIb), Aminonitrilen (IIIc) und Aminoamiden (IIId) zu Amiden (IV), dadurch gekennzeichnet, dass
a) Edukt (III) mit Wasser in der Flüssigphase in Gegenwart eines organischen flüssigen Verdünnungsmittels (V) zu einer Mischung (II) enthaltend Amid (IV) umsetzt, wobei das Verdünnungsmittel (V) mit Wasser eine Mischungslücke unter bestimmten Mengen-, Druck- und Temperaturbedingungen aufweist,
b) Mischung (II) in Mengen-, Druck- und Temperaturbedingungen überführt, unter denen Verdünnungsmittel (V) und Wasser flüssig vorliegen und eine Mischungslücke aufweisen, unter Erhalt eines Zweiphasensystems bestehend aus einer Phase (VII), die einen höheren Anteil an Verdünnungsmittel (V) als an Wasser aufweist, und einer Phase (VIII), die einen höheren Anteil an Wasser als an Verdünnungsmittel (V) aufweist,
c) Phase (VII) von Phase (VIII) abtrennt,
d) in Phase (VII) vorhandenen Ammoniak durch Extraktion (a) mit einer Wasser enthaltenden Mischung (IX) ganz oder teilweise abtrennt unter Erhalt einer den abgetrennten Ammoniak enthaltenden wässrigen Mischung (X) und einer Mischung (XI), die weniger Ammoniak als Phase (VII) enthält und
e) von Mischung (XI) das Verdünnungsmittel (V), gegebenenfalls restlichen Ammoniak und gegebenenfalls Nebenprodukte ausgewählt aus der Gruppe bestehend aus Leichtsieder, Hochsieder und nicht umgesetzter Verbindungen (III) abtrennt unter Erhalt von Amid (IV).

Verfahren zur Herstellung von Amiden, wie cyclischer Lactame, durch Umsetzung von omega-Aminocarbonsäurederivaten, beispielsweise die Herstellung von Caprolactam aus 6-Aminocarbonsäurenitril, mit Wasser in Gegenwart eines heterogenen Katalysators und einem organischen flüssigen Verdünnungsmittel in der Flüssigphase sind allgemein bekannt.

So ist aus WO 95/14665 und WO 95/14664 bekannt, 6-Aminocapronitril in der Flüssigphase mit Wasser in Gegenwart von heterogenen Katalysatoren und einem Lösungsmittel zu Caprolactam und Ammoniak umzusetzen. Die höchsten Caprolactam-Ausbeuten (86 bis 94 %) werden mit Titandioxid als Katalysator und Ethanol als Lösungsmittel erzielt. Die Caprolactam-Ausbeuten wurden dabei nur gaschromatographisch bestimmt; die Aufarbeitung der Reaktorausträge zu Roh- und/oder Reincaprolactam ist nicht beschrieben.

WO 97/23454 beschreibt in Beispiel 1c) die Umsetzung von 6-Aminocapronitril mit Wasser in Gegenwart von Titandioxid und Ethanol. Aus dem Reaktoraustrag wurde durch fraktionierende Destillation Caprolactam in einer Ausbeute von 80 % erhalten.

Nachteilig an der genannten Umsetzung von 6-Aminocapronitril zu Caprolactam in Gegenwart von Ethanol ist der hohe Energieverbrauch hinsichtlich der Abtrennung von Ammoniak aus verdünnten Lösungen.

Die Aufgabe der vorliegenden Erfindung bestand daher darin, ein Verfahren bereitzustellen, das die Abtrennung von Ammoniak aus Mischungen (II), die bei der Umsetzung von Edukten (III) zu Amiden (IV) erhältlich sind, auf technisch einfache und wirtschaftliche Weise ermöglicht und zudem den Energieaufwand bei der Aufarbeitung minimiert.

Demgemäß wurde das eingangs definierte Verfahren gefunden.

Edukte (III) sind erfindungsgemäß ausgewählt aus der Gruppe bestehend aus Nitril en (IIIa), Aminen (IIIb), Aminonitrilen (IIIc) und Aminoamiden (IIId).

Vorteilhaft kommen als Nitril (IIIa) organische Verbindungen mit einer oder mehreren, wie zwei, drei oder vier, vorzugsweise zwei, Nitril-Gruppen, also vorzugsweise Dinitrile, oder Gemische solcher Verbindungen in Betracht.

Als Dinitrile können einzeln oder im Gemisch prinzipiell alle Dinitrile eingesetzt werden. Unter diesen sind die alpha,omega-Dinitrile bevorzugt, wobei unter letzteren insbesondere alpha,omega-Alkylendinitrile mit 3 bis 14 C-Atomen, weiter bevorzugt 3 bis 12 C-Atomen im Alkylenrest, oder ein aromatisches C₈-C₁₂-Dinitril wie Phthalodinitril, Isophthalodinitril oder Terephtalodinitril, sowie ein C₅-C₈-Cycloalkandinitril wie Cyclohexandinitril eingesetzt werden.

Als alpha,omega-Dinitril setzt man weiter bevorzugt lineare alpha,omega-Dinitrile ein, wobei der Alkylenrest (-CH₂-) vorzugsweise 2 bis 14, weiter bevorzugt 3 bis 12 C-Atome enthält, wie Ethan-1,2-dinitril (Bernsteinsäuredinitril), Propan-1,3-dinitril (Glutarsäuredinitril), Butan-1,4-dinitril (Adipodinitril), Pentan-1,5-dinitril (Pimelinsäuredinitril), Hexan-1,6-dinitril (Korksäuredinitril), Heptan-1,7-dinitril (Azelainsäuredinitril), Octan-1,8-dinitril (Sebazinsäurediniril), Nonan-1,9-dinitril, Decan-1,10-dinitril, besonders bevorzugt Adipodinitril.

Adipodinitril kann nach an sich bekannten Verfahren durch doppelte Hydrocyanierung von Butadien erhalten werden.

Selbstverständlich können auch Gemische mehrerer Nitrile mit gleicher oder unterschiedlicher Anzahl an Nitril-Gruppen, insbesondere mehrerer Dinitrile, eingesetzt werden.

Gewünschtenfalls kann man auch Dinitrile, die sich von verzweigten Alkylen- oder Arylen- oder Alkylarylenen ableiten, verwenden.

Vorteilhaft kommen als Amin (IIIb) organische Verbindungen mit einer oder mehreren, wie zwei, drei oder vier, vorzugsweise zwei, Amino-Gruppen, also vorzugsweise Diamine, oder Gemische solcher Verbindungen in Betracht.

Als Diamin können einzeln oder im Gemisch prinzipiell alle Diamine eingesetzt werden, wie aromatische Amine, beispielsweise 1,4-Phenylendiamin oder 4,4'-Diaminodiphenylpropan, oder aliphatische Amine. Unter diesen sind die alpha,omega-Diamine bevorzugt, wobei unter letzteren insbesondere alpha,omega-Alkylendiamine mit 3 bis 14 C-Atomen, weiter bevorzugt 3 bis 10 C-Atomen im Alkylenrest, oder Alkylaryldiamine mit 9 bis 14 C-Atomen im Alkylrest eingesetzt werden, wobei dort solche bevorzugt werden, die zwischen der aromatischen Einheit und den beiden Aminogruppen eine Alkylengruppe mit mindestens einem C-Atom aufweisen, wie p-Xylylendiamin oder bevorzugt m-Xylylendiamin.

Als alpha,omega-Diamine setzt man weiter bevorzugt lineare alpha,omega-Diamine ein, wobei der Alkylenrest (-CH₂-) vorzugsweise 3 bis 14, weiter bevorzugt 3 bis 10 C-Atome enthält, wie 1,3-Diaminopropan, 1,4-Diaminobutan, 1,5-Diaminopentan, 1,6-Diaminohexan (Hexamethylendiamin, HMD), 1,7-Diaminoheptan, 1,8-Diaminooctan, 1,9-Diaminononan, 1,10-Diaminodecan, besonders bevorzugt Hexamethylendiamin.

Hexamethylendiamin kann nach an sich bekannten Verfahren durch doppelte katalytische Hydrierung der Nitrilgruppen von Adipodinitril erhalten werden.

Selbstverständlich können auch Gemische mehrerer Diamine eingesetzt werden.

Gewünschtenfalls kann man auch Diamine, die sich von verzweigten Alkylen- oder Arylen- oder Alkylarylenen ableiten, verwenden, wie 2-Methyl-1,5-Diaminopentan.

Vorteilhaft kommen als Aminonitril (IIIc) organische Verbindungen mit einer oder mehreren, wie zwei, drei oder vier, vorzugsweise einer, Amino-Gruppe und einer oder mehreren, wie zwei, drei oder vier, vorzugsweise einer, Nitril-Gruppe, also vorzugsweise Monoamino-mononitrile ("Aminocarbonsäurenitrile"), oder Gemische solcher Verbindungen in Betracht.

Unter den Aminocarbonsäurenitrilen sind die omega-Aminocarbonsäurenitrile bevorzugt, wobei unter letzteren insbesondere omega-Aminocarbonsäurenitrile mit 3 bis 12 C-Atomen, weiter bevorzugt 3 bis 9 C-Atomen im Alkylenrest, oder Aminoalkylarylcarbonsäurenitrile mit 7 bis 13 C-Atomen im Alkylenrest eingesetzt werden, wobei dort solche bevorzugt werden, die zwischen der aromatischen Einheit und der Amino- und Nitrilgruppe eine Alkylengruppe mit mindestens einem C-Atom aufweisen. Unter den Aminoalkylarylcarbonsäurenitrilen sind insbesondere solche bevorzugt, die die Amino- und Nitrilgruppe in 1,4-Stellung zueinander aufweisen.

Als omega-Aminocarbonsäurenitril setzt man weiter bevorzugt lineare omega-Aminocarbonsäurenitrile ein, wobei der Alkylenrest (-CH₂-) vorzugsweise 3 bis 14, weiter bevorzugt 3 bis 9 C-Atome enthält, wie 3-Amino-1-nitrilo-propan, 4-Amino-1-nitril-butan, 5-Amino-1-nitrilo-pentan (6-Aminocapronitril), 6-Amino-1-nitrilohexan, 7-Amino-1-nitrilo-heptan, 8-Amin-1-nitrilo-octan, 9-Amino-1-nitrilo-nonan, besonders bevorzugt 6-Aminocapronitril.

6-Aminocapronitril kann nach an sich bekannten Verfahren durch einfache katalytische Hydrierung einer der Nitrilgruppen von Adipodinitril erhalten werden.

Selbstverständlich können auch Gemische mehrerer Aminocarbonsäurenitrile eingesetzt werden.

Gewünschtenfalls kann man auch Aminocarbonsäurenitrile, die sich von verzweigten Alkylen- oder Arylen- oder Alkylarylenen ableiten, verwenden.

Vorteilhaft kommen als Aminoamid (IIId) organische Verbindungen mit einer oder mehreren, wie zwei, drei oder vier, vorzugsweise einer, Amino-Gruppe und einer oder mehreren, wie zwei, drei oder vier, vorzugsweise einer, Carboamid-Gruppe (-CONH₂), also vorzugsweise Monoamino-monoamide ("Aminocarbonsäureamide"), oder Gemische solcher Verbindungen in Betracht.

Unter den Aminocarbonsäureamiden sind die omega-Aminocarbonsäureamide bevorzugt, wobei unter letzteren insbesondere omega-Aminocarbonsäureamide mit 3 bis 12 C-Atomen, weiter bevorzugt 3 bis 9 C-Atomen im Alkylenrest, oder Aminoalkylarylcarbonsäureamide mit 7 bis 13 C-Atomen im Alkylenrest eingesetzt werden, wobei dort solche bevorzugt werden, die zwischen der aromatischen Einheit und der Amino- und Carboamidgruppe eine Alkylengruppe mit mindestens einem C-Atom aufweisen. Unter den Aminoalkylarylcarbonsäureamiden sind insbesondere solche bevorzugt, die die Amino- und Carboamidgruppe in 1,4-Stellung zueinander aufweisen.

Als omega-Aminocarbonsäureamide setzt man weiter bevorzugt lineare omega-Aminocarbonsäureamide ein, wobei der Alkylenrest (-CH₂-) vorzugsweise 3 bis 14, weiter bevorzugt 3 bis 9 C-Atome enthält, wie 3-Amino-1-carbamido-propan, 4-Amino-1-carbamido-butan, 5-Amino-1-carbamido-pentan (6-Aminocapronsäureamid), 6-Amino-1-carbamido-hexan, 7-Amino-1-carbamido-heptan, 8-Amin-1-carbamido-octan, 9-Amino-1-carbamido-nonan, besonders bevorzugt 6-Aminocapronsäureamid.

6-Aminocapronsäureamid kann nach an sich bekannten Verfahren durch partielle Hydrolyse der Nitrilgruppe von 6-Aminocapronitril erhalten werden.

Selbstverständlich können auch Gemische mehrerer Aminocarbonsäureamide eingesetzt werden.

Gewünschtenfalls kann man auch Aminocarbonsäureamide, die sich von verzweigten Alkylen- oder Arylen- oder Alkylarylenen ableiten, verwenden.

Es können auch Gemische von Verbindungen (IIIa), (IIIb), (IIIc) und (IIId) eingesetzt werden.

Edukt (III) kann neben Verbindungen (IIIa), (IIIb), (IIIc) und (IIId) weitere Verbindungen, die zur Bildung der Amidgruppen von (IV) fähige funktionelle Gruppen aufweisen, wie Carbonsäuregruppen, Carbonsäureestergruppen oder Lactame, beispielsweise Adipinsäure oder Caprolactam, enthalten.

Enthält Edukt (III) Nitril (IIIa) und Amin (IIIb), enthält beispielsweise Edukt (III) Adipodinitril und Hexamethylendiamin in Gegenwart oder Abwesenheit von Verbindungen (IIIc) und/oder (IIId), so sollte das molare Verhältnis der zur Bildung der Amidgruppen von (IV) herangezogenen Nitril-Gruppen von (IIIa) zu den zur Bildung der Amidgruppen von (IV) herangezogenen Amin-Gruppen von (IIIb) vorteilhaft zwischen 0,8 und 1,2, vorzugsweise zwischen 0,95 und 1,05, besonders bevorzugt zwischen 0,98 und 1,02 (äquimolar) betragen.

In Schritt a) des erfindungsgemäßen Verfahrens erhält man ein Amid (IV), ausgewählt aus der Gruppe bestehend aus Lactam (IVa), Oligomer (IVb) und Polymer (IVc) mit Amidgruppen in der Hauptkette.

Lactame (IVa) können vorteilhaft aus solchen Edukten erhalten werden, die zur Bildung einer inneren Amidgruppe mit sich selbst fähig sind, vorzugsweise aus (IIIc) und (IIId). Die Struktur der Lactame (IVa) ergibt sich dabei unmittelbar aus der Struktur der Edukte (III).

Unter Oligomere (IVb) werden im Sinne der vorliegenden Erfindung Verbindungen verstanden, die durch Verknüpfung von wenigen, wie zwei, drei, vier, fünf oder sechs, Molekülen, ausgewählt aus der Gruppe der als Edukt (III) eingesetzten verbindungen, über Amidfunktionen entstehen, wie Dimere, Trimere, Tetramere, Pentamere oder Hexamere von 6-Aminocapronitril oder 6-Aminocaproamid oder einer Adipodinitril/Hexamethylendiamin-Mischung oder deren Gemische.

Unter Polymere (IVc) werden im Sinne der vorliegenden Erfindung hochmolekulare Verbindungen verstanden, die in der Hauptkette wiederkehrende Amidgruppen (-CONH-) aufweisen, beispielsweise Polycaprolactam (Nylon 6) oder Poly(hexamethylenammoniumadipat) (Nylon 6.6).

In dem erfindungsgemäßen Verfahren setzt man in Schritt a) die vorstehend beschriebenes Edukt (III) mit Wasser in der Flüssigphase, vorzugsweise in einer homogenen flüssigen Phase, vorteilhaft in Gegenwart eines heterogenen Katalysators und eines organischen flüssigen Verdünnungsmittel (V) zu einer Mischung (II) enthaltend ein Amid (IV) um, wobei das Verdünnungsmittel (V) mit Wasser eine Mischungslücke unter bestimmten Mengen-, Druck- und Temperaturbedingungen aufweist.

Als heterogene Katalysatoren sind saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titandioxid, amorph, als Anatas oder Rutil, Zirkondioxid, Manganoxid oder Mischungen davon geeignet. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:
Vanadiumoxid, Bariumoxid, Zinkoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. dieses enthalten.

Weiterhin sind Zeolithe, Phosphate und Heteropolysäuren, sowie saure und alkalische Ionenaustauscher wie beispielsweise Nafion als geeignete Katalysatoren zu nennen.

Bevorzugte Katalysatoren sind Titanoxid, Aluminiumoxid, Ceroxid und Zirkondioxid, besonders bevorzugte Katalysatoren sind Titandioxide, wie sie beispielsweise aus WO 96/36600 bekannt sind. Die Herstellung solcher Katalysatoren als Formkörper ist beispielsweise in WO 99/11613, WO 99/11614 und WO 99/11615 beschrieben.

Als Verdünnungsmittel (V) kommen C₄- bis C₉-Alkanole, wie n-Butanol, i-Butanol, n-Pentanol, vorzugsweise aliphatische Kohlenwasserstoffe, wie n-Hexan, cycloaliphatische Kohlenwasserstoffe, wie Cyclopentan oder Cyclohexan, besonders bevorzugt aromatische Kohlenwasserstoffe, wie Benzol, Toluol, o-Xylol, m-Xylol, p-Xylol, Ethylbenzol, i-Propylbenzol, Di-i-propylbenzol, insbesondere Benzol, Toluol, o-Xylol, m-Xylol, p-Xylol, Ethylbenzol, sowie Gemische solcher Verbindungen, beispielsweise Petrolether, in Betracht. Die Kohlenwasserstoffe können funktionelle Gruppen, wie Halogene, beispielsweise Chlor, tragen, wie in Chlorbenzol.

Bei der Umsetzung in Schritt a) sollten im allgemeinen pro mol Verbindung (III) mindestens 1 mol, vorzugsweise 2 bis 100, insbesondere 2 bis 10 mol Wasser eingesetzt werden.

Vorteilhaft beträgt in Schritt a) der Anteil der Verbindung (III) an der Summe der Ausgangskomponenten Verbindung (III), Wasser und Verdünnungsmittel (V) 0,1 bis 50 Gew.-%, vorzugsweise 1 bis 30 Gew.-%, insbesondere 2 bis 20 Gew.-%.

Die Umsetzung kann vorteilhaft in flüssiger Phase bei Temperaturen von im allgemeinen 140 bis 320°C, vorzugsweise 180 bis 300°C, besonders bevorzugt 200 bis 280°C durchgeführt werden. Der Druck sollte im allgemeinen im Bereich von 1 bis 250 bar, vorzugsweise von 5 bis 150 bar liegen.

Dabei sind solche Druck- und Temperaturbedingungen bevorzugt, unter denen das Reaktionsgemisch in einer einzigen homogenen flüssigen Phase vorliegt.

Die Katalysatorbelastungen liegen im allgemeinen im Bereich von 0,05 bis 5, vorzugsweise 0,1 bis 2, insbesondere 0,2 bis 1 kg Reaktionsmischung / 1 Katalysatorvolumen / Stunde.

Bei der Umsetzung gemäß Schritt a) wird eine Mischung (II) erhalten, die ein Amid (IV), Ammoniak (I) und gegebenenfalls Nebenprodukte ausgewählt aus der Gruppe bestehend aus Leichtsieder, Hochsieder und nicht umgesetzt Verbindung (III), enthält.

Unter Leichsieder werden dabei im Sinne der vorliegenden Erfindung Verbindungen mit einem Siedepunkt unterhalb dessen von Amid (IV) verstanden, unter Hochsieder (VII) solche Verbindungen mit einem Siedepunkt oberhalb dessen von Amid (IV).

Gemäß Schritt b) wird erfindungsgemäß Mischung (II) in Mengen-, Druck- und Temperaturbedingungen überführt, unter denen Verdünnungsmittel (V) und Wasser flüssig vorliegen und eine Mischungslücke aufweisen, unter Erhalt eines Zweiphasensystems aus einer Phase (VII), die einen höheren Anteil an Verdünnungsmittel (V) als an Wasser aufweist, und einer Phase (VIII), die einen höheren Anteil an Wasser als an Verdünnungsmittel (V) aufweist.

Bevorzugt sind solche Mengen-, Druck- und Temperaturbedingungen, unter denen die Bestandteile der Mischung (II) in den Phasen (VII) und (VIII) vollständig flüssig vorliegen, also keine Feststoffe ausfallen.

Wurde Schritt a) in einer homogenen Flüssigphase durchgeführt, so kann im allgemeinen eine Auftrennung der Mischung (II) in die beiden Phasen (VII) und (VIII) durch Wahl einer geeigneten Temperatur erreicht werden. Als weitere Möglichkeit kommt die Wahl geeigneter Mengenverhältnisse in Betracht wie der Zusatz von Verdünnungsmittel (V), vorzugsweise von Wasser.

Anschließend werden erfindungsgemäß Phase (VII) und Phase (VIII) gemäß Schritt c) getrennt.

Die Phasentrennung kann in an sich bekannter Weise in für solche Zwecke beschriebenen Apparaturen erfolgen, wie sie beispielsweise aus: Ullmann's Encyclopedia of Industrial Chemistry, Vol. B3, 5. Ed., VCH Verlagsgesellschaft, Weinheim, 1988, Seite 6-14 bis 6-22 bekannt sind, wie Dekanter, Zyklone oder Zentrifugen.

Die für die Phasentrennung optimalen Apparaturen und Verfahrensbedingungen lassen sich dabei leicht durch einige einfache Vorversuche ermitteln.

Gemäß Schritt d) werden erfindungsgemäß in Phase (VII) vorhandener Ammoniak durch Extraktion (a) mit einer Wasser enthaltenden Mischung (IX) ganz oder teilweise abgetrennt unter Erhalt einer den abgetrennten Ammoniak enthaltenden wäßrigen Mischung (X) und einer Mischung (XI), die weniger Ammoniak enthält als Phase (VII).

Als Mischung (IX) kann vorteilhaft Wasser, eine nachfolgend definierte Mischung (XIII) ganz oder teilweise, eine nachfolgend definierte Mischung (XIV), deren Wassergehalt höher als der von Mischung (XIII) ist, ganz oder teilweise oder deren Gemische eingesetzt werden.

Die Extraktion (a) kann in an sich bekannter Weise in für solche Zwecke beschriebenen Apparaturen erfolgen, wie sie beispielsweise aus: Ullmann's Encyclopedia of Industrial Chemistry, Vol. B3, 5. Ed., VCH Verlagsgesellschaft, Weinheim, 1988, Seite 6-14 bis 6-22 bekannt sind, wie Sieb- oder Füllkörperkolonnen, pulsiert oder nicht pulsiert, oder Mixer-Settler.

Die für die Extraktion (a) optimalen Apparaturen und Verfahrensbedingungen lassen sich dabei leicht durch einige einfache Vorversuche ermitteln.

Gemäß Schritt e) werden erfindungsgemäß von Mischung (XI) Verdünnungsmittel (V) und gegebenenfalls restlicher Ammoniak und gegebenenfalls Nebenprodukte ausgewählt aus der Gruppe bestehend aus Leichtsieder, Hochsieder und nicht umgesetzter Verbindung (III) abgetrennt unter Erhalt von Amid (IV).

Unter Leichtsieder werden dabei im Sinne der vorliegenden Erfindung Verbindungen mit einem Siedepunkt unterhalb dessen von Amid (IV) verstanden, unter Hochsieder solche Verbindungen mit einem Siedepunkt oberhalb dessen von Amid (IV).

Diese Aufarbeitung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Vorteilhaft kann man aus Phase (VIII), vorzugsweise aus Phase (VIII) und Mischung (X) gemeinsam, Ammoniak vollständig oder teilweise durch Destillation (b1) oder Rektifikation (b2) abtrennen unter Erhalt einer Mischung (XII), die den wesentlichen Teil des Ammoniaks enthält und einer Mischung (XIII), in der der Ammoniakgehalt geringer ist als in Phase (VIII).

Vorzugsweise kommt dabei eine destillative Abtrennung (b1) oder (b2) von Ammoniak bei einem Druck von weniger als 8 bar absolut in Betracht, wobei insbesondere der Ammoniak dampfförmig abgezogen wird.

Diese Aufarbeitung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen, insbesondere einer Kolonne mit Seitenabzug.

Bei einer Kolonne mit Seitenabzug kann dabei eine Mischung (XIV) an einem Seitenabzug der bei der Destillation (b1) oder Rektifikation (b2) eingesetzten Vorrichtung erhalten werden.

Der dampfförmig abgezogene Ammoniak kann vorteilhaft einer Behandlung (c) mit einem Alkali (XV) unterzogen werden unter Erhalt eines gereinigten Ammoniaks (XVI). Als Alkali (XV) kommen basisch reagierende Verbindungen, vorzugsweise Oxide und Hydroxide, insbesondere solche der ersten und zweiten Hauptgruppe, wie Natriumhydroxid, in Betracht.

Diese Aufarbeitung kann vorteilhaft durch Wäsche in einer oder mehreren, wie 2 oder 3 Apparaturen erfolgen, vortielhaft durch Gegenstromführung von Ammoniak (XII) und Waschmittel (XV).

Dabei kommen für die Wäsche hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen, Venturi-Wäscher oder Sprühkolonnen.

In einer vorteilhaften Ausführungsform kann Mischung (XII) oder Ammoniak (XVI) in Wasser absorbiert werden (d) unter Erhalt einer wäßrigen Mischung (XVII), die Ammoniak enthält.

In einer weiteren vorteilhaften Ausführungsform kann Mischung (XII) oder Ammoniak (XVI) auf einen höheren Druck verdichtet werden unter Erhalt einer Mischung (XVIII).

Mischung (XII) oder Mischung (XIII) können bei einem Druck von mehr als 8 bar absolut destilliert werden unter Erhalt einer Mischung (XIX), die weniger Wasser und weniger Verdünnungsmittel (V) als Mischung (XVIII) enthält, und einer Mischung (XX), die weniger Ammoniak als Mischung (XVIII) enthält.

Mischung (XX) kann vorteilhaft in Absorption (d) teilweise oder vollständig eingesetzt werden.

Aus Mischung (XX) kann vorteilhaft Verdünnungsmittel (V) abgetrennt und in Schritt a) des erfindungsgemäßen Verfahrens zurückgeführt werden.

In einer weiteren vorteilhaften Ausführungsform kann Mischung (XIII) teilweise oder vollständig in Stufe a) des erfindungsgemäßen Verfahrens zurückgeführt werden.

Die nach dem erfindungsgemäßen Verfahren erhältlichen Amide (IV) sind wertvolle Zwischenstufen bei der Herstellung technisch bedeutsamer Polymere, insbesondere Polyamide. Solche Polyamide oder auch Polymer (IVc) können zur Herstellung von Fasern, Folien und Formkörpern in an sich bekannter Weise eingesetzt werden.

## Patentansprüche

1. Verfahren zur Abtrennung von Ammoniak (I) aus Mischungen (II) enthaltend Ammoniak (I) und ein Amid (IV), ausgewählt aus der Gruppe bestehend aus Lactam (IVa), Oligomer (IVb) und Polymer (IVc) mit Amidgruppen in der Hauptkette, wobei Amid (IV) erhalten wurde durch Umsetzung von Edukten (III) ausgewählt aus der Gruppe bestehend aus Nitrilen (IIIa), Aminen (IIIb), Aminonitrilen (IIIc) und Aminoamiden (IIId) mit Wasser, **dadurch gekennzeichnet, dass** man
a) Edukt (III) mit Wasser in der Flüssigphase in Gegenwart eines organischen flüssigen Verdünnungsmittels (V) zu einer Mischung (II) enthaltend Amid (IV) und Ammoniak (I) umsetzt, wobei das Verdünnungsmittel (V) mit Wasser eine Mischungslücke unter bestimmten Mengen-, Druck- und Temperaturbedingungen aufweist,
b) Mischung (II) in Mengen-, Druck- und Temperaturbedingungen überführt, unter denen Verdünnungsmittel (V) und Wasser flüssig vorliegen und eine Mischungslücke aufweisen, unter Erhalt eines Zweiphasensystems bestehend aus einer Phase (VII), die einen höheren Anteil an Verdünnungsmittel (V) als an Wasser aufweist, und einer Phase (VIII), die einen höheren Anteil an Wasser als an Verdünnungsmittel (V) aufweist,
c) Phase (VII) von Phase (VIII) abtrennt,
d) in Phase (VII) vorhandenen Ammoniak durch Extraktion (a) mit einer Wasser enthaltenden Mischung (IX) ganz oder teilweise abtrennt unter Erhalt einer den abgetrennten Ammoniak enthaltenden wässrigen Mischung (X) und einer Mischung (XI), die weniger Ammoniak als Phase (VII) enthält und
e) von Mischung (XI) Verdünnungsmittel (V), gegebenenfalls restlichen Ammoniak und gegebenenfalls Nebenprodukte ausgewählt aus der Gruppe bestehend aus Leichtsieder, Hochsieder und nicht umgesetzter Verbindungen (III) abtrennt unter Erhalt von Amid (IV).

2. Verfahren nach Anspruch 1, wobei aus Phase (VIII) Ammoniak vollständig oder teilweise durch Destillation (b1) oder Rektifikation (b2) abgetrennt wird unter Erhalt einer Mischung (XII), die im wesentlichen Ammoniak enthält und einer Mischung (XIII), in der der Ammoniakgehalt geringer als in Phase (VIII) ist.

3. Verfahren nach Anspruch 1 oder 2, wobei man Phase (VIII) und Mischung (X) in Destillation (b1) oder Rektifikation (b2) zusammen aufarbeitet und Ammoniak abtrennt.

4. Verfahren nach den Ansprüchen 1 bis 3, wobei als wässrige Mischung (IX) Mischung (XIII) vollständig oder teilweise eingesetzt wird.

5. Verfahren nach den Ansprüchen 1 bis 4, wobei als wässrige Mischung (IX) eine Mischung (XIV) eingesetzt wird, dessen Wassergehalt höher als der von Mischung (XIII) ist.

6. Verfahren nach Anspruch 5, wobei Mischung (XIV) an einem Seitenabzug der bei der Destillation (b1) oder Rektifikation (b2) eingesetzten Vorrichtung erhalten wird.

7. Verfahren nach den Ansprüchen 1 bis 6, wobei Mischung (XIII) teilweise oder vollständig zum Reaktor zur Synthese von Amid (IV) aus Edukt (III) zurückgeführt wird.

8. Verfahren nach den Ansprüchen 2 bis 7, wobei die destillative Abtrennung (b1) oder (b2) von Ammoniak bei einem Druck von weniger als 8 bar absolut durchgeführt wird und Ammoniak dampfförmig abgezogen wird.

9. Verfahren nach Anspruch 8, wobei der dampfförmig abgezogene Ammoniak einer Behandlung (c) mit einem Alkali (XV) unterzogen wird unter Erhalt eines gereinigten Ammoniaks (XVI).

10. Verfahren nach Anspruch 9., wobei als Alkali (XV) NaOH eingesetzt wird.

11. Verfahren nach den Ansprüchen 1 bis 10, wobei Mischung (XII) oder Ammoniak (XVI) in Wasser absorbiert (d) wird unter Erhalt einer wässrigen Mischung (XVII), die Ammoniak enthält.

12. Verfahren nach den Ansprüchen 1 bis 10, wobei Mischung (XII) oder Ammoniak (XVI) auf einen höheren Druck verdichtet wird unter Erhalt einer Mischung (XVIII).

13. Verfahren nach Anspruch 11 oder 12, wobei Mischung (XVII) oder Mischung (XVIII), bei einem Druck von mehr als 8 bar absolut destilliert wird unter Erhalt einer Mischung (XIX), die weniger Wasser und weniger Verdünnungsmittel (V) als Mischung (XVIII) enthält und einer Mischung (XX), die weniger Ammoniak als Mischung (XVII) oder Mischung (XVIII) enthält.

14. Verfahren nach Anspruch 11 oder 13, wobei Mischung (XX) vollständig oder teilweise zur Absorption (d) verwendet wird.

15. Verfahren nach Anspruch 13 oder 14, wobei aus Mischung (XX) Verdünnungsmittel (V) abgetrennt wird und in die Synthese von Amid (IV) aus Edukt (III) zurückgeführt wird.

16. Verfahren nach den Ansprüchen 1 bis 15, wobei 6-Aminocapronitril als Aminonitril (IIIc) eingesetzt wird.

17. Verfahren nach den Ansprüchen 1 bis 16, wobei Adipodinitril als Nitril (IIIa) eingesetzt wird.

18. Verfahren nach den Ansprüchen 1 bis 17, wobei Hexamethylendiamin als Amin (IIIb) eingesetzt werden.

19. Verfahren nach den Ansprüchen 1 bis 18, wobei ein Verdünnungsmittel (V) ausgewählt aus der Gruppe bestehend aus Ethylbenzol, Benzol, Toluol, o-Xylol, m-Xylol und p-Xylol eingesetzt wird.

## Claims

1. A process for the separation of ammonia (I) from mixtures (II) containing ammonia (I) and an amide (IV) selected from the group consisting of a lactam (IVa), an oligomer (IVb) and a polymer (IVc) with amide groups in the main chain, said amide (IV) having been obtained by reacting educts (III), selected from the group consisting of nitriles (IIIa), amines (IIIb), amino nitriles (IIIc) and amino amides (IIId), with water, wherein
a) the educt (III) is reacted with water in the liquid phase, in the presence of an organic liquid diluent (V), to give a mixture (II) containing the amide (IV) and the ammonia (I), the diluent (V) exhibiting a miscibility gap with water under certain quantity, pressure and temperature conditions,
b) the mixture (II) is converted under quantity, pressure and temperature conditions such that the diluent (V) and the water are in liquid form and exhibit a miscibility gap, to give a two-phase system consisting of a phase (VII) containing a higher proportion of diluent (V) than water, and a phase (VIII) containing a higher proportion of water than diluent (V),
c) the phase (VII) is separated from the phase (VIII),
d) all or part of the ammonia present in the phase (VII) is separated off by extraction (a) with a water-containing mixture (IX) to give an aqueous mixture (X) containing the ammonia which has been separated off, and a mixture (XI) containing less ammonia than the phase (VII), and
e) the diluent (V), any residual ammonia and any by-products selected from the group consisting of low-boiling components, high-boiling components and unreacted compounds (III) are separated from the mixture (XI) to give the amide (IV).

2. A process as claimed in claim 1 wherein all or part of the ammonia is separated from the phase (VIII) by distillation (b1) or rectification (b2) to give a mixture (XII) containing essentially ammonia, and a mixture (XIII) in which the ammonia content is less than that of the phase (VIII).

3. A process as claimed in claim 1 or 2 wherein the phase (VIII) and the mixture (X) are worked up together in the distillation (b1) or the rectification (b2) and the ammonia is separated off.

4. A process as claimed in any of claims 1 to 3 wherein all or part of the mixture (XIII) is used as the aqueous mixture (IX).

5. A process as claimed in any of claims 1 to 4 wherein a mixture (XIV) in which the water content is greater than that of the mixture (XIII) is used as the aqueous mixture (IX).

6. A process as claimed in claim 5 wherein the mixture (XIV) is obtained at a side discharge of the device used in the distillation (b1) or the rectification (b2).

7. A process as claimed in any of claims 1 to 6 wherein all or part of the mixture (XIII) is recycled into the reactor for synthesizing the amide (IV) from the educt (III).

8. A process as claimed in any of claims 2 to 7 wherein the distillative separation (b1) or (b2) of the ammonia is carried out at a pressure of less than 8 bar absolute and the ammonia is withdrawn in the vapor state.

9. A process as claimed in claim 8 wherein the ammonia withdrawn in the vapor state is subjected to a treatment (c) with an alkali (XV) to give a purified ammonia (XVI).

10. A process as claimed in claim 9 wherein NaOH is used as the alkali (XV).

11. A process as claimed in any of claims 1 to 10 wherein the mixture (XII) or the ammonia (XVI) is absorbed in water, (d), to give an aqueous mixture (XVII) containing ammonia.

12. A process as claimed in any of claims 1 to 10 wherein the mixture (XII) or the ammonia (XVI) is compressed to a higher pressure to give a mixture (XVIII).

13. A process as claimed in claim 11 or 12 wherein the mixture (XVII) or the mixture (XVIII) is distilled at a pressure of more than 8 bar absolute to give a mixture (XIX) containing less water and less diluent (V) than the mixture (XVIII), and a mixture (XX) containing less ammonia than the mixture (XVII) or the mixture (XVIII).

14. A process as claimed in claim 11 or 13 wherein all or part of the mixture (XX) is used for the absorption (d).

15. A process as claimed in claim 13 or 14 wherein the diluent (V) is separated from the mixture (XX) and recycled into the synthesis of the amide (IV) from the educt (III).

16. A process as claimed in any of claims 1 to 15 wherein 6-aminocapronitrile is used as the amino nitrile (IIIc).

17. A process as claimed in any of claims 1 to 16 wherein adipodinitrile is used as the nitrile (IIIa).

18. A process as claimed in any of claims 1 to 17 wherein hexamethylenediamine is used as the amine (IIIb).

19. A process as claimed in any of claims 1 to 18 wherein a diluent (V) selected from the group consisting of ethylbenzene, benzene, toluene, o-xylene, m-xylene and p-xylene is used.

## Revendications

1. Procédé de séparation d'ammoniac (I) à partir de mélanges (II) contenant de l'ammoniac (I) et un amide (IV), choisi parmi le groupe constitué d'un lactame (IVa), d'un oligomère (IVb) et d'un polymère (IVc) présentant des groupes amide dans la chaîne principale, dans lequel l'amide (IV) est obtenu par réaction de matières de départ (III) choisies parmi le groupe constitué de nitriles (IIIa), d'amines (IIIb), d'aminonitriles (IIIc) et d'aminoamides (IIId) avec de l'eau, **caractérisé en ce que**
a) on fait réagir de la matière de départ (III) avec de l'eau dans la phase liquide en présence d'un diluant liquide organique (V) pour former un mélange (II) contenant de l'amide (IV) et de l'ammoniac (I), le diluant (V) présentant avec l'eau une lacune de miscibilité dans des conditions de quantité, de pression et de température déterminées,
b) on fait passer le mélange (II) dans des conditions de quantité, de pression et de température dans lesquelles le diluant (V) et l'eau se présentent sous forme liquide et présentent une lacune de miscibilité, avec obtention d'un système à deux phases constitué d'une phase (VII), qui présente une fraction plus élevée en diluant (V) qu'en eau et d'une phase (VIII) qui présente une fraction plus élevée d'eau que de diluant (V),
c) on sépare la phase (VII) de la phase (VIII),
d) on sépare totalement ou partiellement l'ammoniac présent dans la phase (VII) par extraction (a) avec un mélange contenant de l'eau (IX), avec obtention d'un mélange aqueux (X) contenant l'ammoniac séparé et d'un mélange (XI), qui contient moins d'ammoniac que la phase (VII), et
e) du mélange (XI) on sépare du diluant (V), éventuellement de l'ammoniac résiduaire et éventuellement des sous-produits choisis parmi le groupe constitué de substances à bas point d'ébullition, de substances à haut point d'ébullition et de composés (III) n'ayant pas réagi, avec obtention d'amide (IV).

2. Procédé suivant la revendication 1, dans lequel on sépare de la phase (VIII) de l'ammoniac totalement ou partiellement par distillation (b1) ou par rectification (b2), avec obtention d'un mélange (XII) qui contient essentiellement de l'ammoniac, et d'un mélange (XIII), dans lequel la teneur en ammoniac est plus faible que dans la phase (VIII).

3. Procédé suivant l'une des revendications 1 et 2, dans lequel on traite la phase (VIII) et le mélange (X) ensemble dans une distillation (b1) ou une rectification (b2) et on isole de l'ammoniac.

4. Procédé suivant les revendications 1 à 3, dans lequel, comme mélange aqueux (IX), on met en oeuvre complètement ou partiellement le mélange (XIII).

5. Procédé suivant les revendications 1 à 4, dans lequel, comme mélange aqueux (IX), on met en oeuvre un mélange (XIV) dont la teneur en eau est plus élevée que celle du mélange (XIII).

6. Procédé suivant la revendication 5, dans lequel le mélange (XIV) est obtenu à un soutirage latéral du dispositif mis en oeuvre lors de la distillation (b1) ou de la rectification (b2).

7. Procédé suivant les revendications 1 à 5, dans lequel le mélange (XIII) est recyclé partiellement ou totalement au réacteur de synthèse d'amide (IV) à partir de la matière de départ (III).

8. Procédé suivant les revendications 2 à 7, dans lequel la séparation par distillation (b1) ou (b2) d'ammoniac est effectuée à une pression inférieure à 8 bars en absolu et l'ammoniac est soutiré sous forme de vapeur.

9. Procédé suivant la revendication 8, dans lequel l'ammoniac soutiré sous forme de vapeur est soumis à un traitement (c) avec un alcali (XV), avec obtention d'ammoniac purifié (XVI).

10. Procédé suivant la revendication 9, dans lequel on met en oeuvre, comme alcali (XV), NaOH.

11. Procédé suivant les revendications 1 à 10, dans lequel le mélange (XII) ou l'ammoniac (XVI) est absorbé dans l'eau (d) avec obtention d'un mélange aqueux (XVII) qui contient de l'ammoniac.

12. Procédé suivant les revendications 1 à 10, dans lequel le mélange (XII) ou l'ammoniac (XVI) est comprimé à une pression supérieure, avec obtention d'un mélange (XVIII).

13. Procédé suivant l'une des revendications 11 et 12, dans lequel le mélange (XVII) ou le mélange (XVIII) est distillé à une pression supérieure à 8 bars en absolu, avec obtention d'un mélange (XIX) qui contient moins d'eau et moins de diluant (V) que le mélange (XVIII) et d'un mélange (XX) qui contient moins d'ammoniac que le mélange (XVII) ou le mélange (XVIII).

14. Procédé suivant l'une des revendications 11 et 13, dans lequel le mélange (XX) est utilisé complètement ou partiellement pour l'absorption (d).

15. Procédé suivant l'une des revendications 13 et 14, dans lequel, à partir du mélange (XX), on sépare du diluant (V) et on le recycle dans la synthèse d'amide (IV) à partir de la matière de départ (III).

16. Procédé suivant les revendications 1 à 15, dans lequel du 6-aminocapronitrile est mis en oeuvre comme aminonitrile (IIIc).

17. Procédé suivant les revendications 1 à 16, dans lequel de l'adipodinitrile est mis en oeuvre comme nitrile (IIIa).

18. Procédé suivant les revendications 1 à 17, dans lequel de l'hexaméthylènediamine est mise en oeuvre comme amine (IIIb).

19. Procédé suivant les revendications 1 à 18, dans lequel on met en oeuvre un diluant (V) choisi parmi le groupe constitué de l'éthylbenzène, du benzène, du toluène, du o-xylène, du m-xylène et du p-xylène.
